(19) **Europäisches Patentamt**

**European Patent Office**

**Office européen des brevets**

(11) Veröffentlichungsnummer: **0 310 542 B1**

(12) **EUROPÄISCHE PATENTSCHRIFT**

(45) Veröffentlichungstag der Patentschrift: **08.06.94**

(21) Anmeldenummer: **88730222.2**

(22) Anmeldetag: **30.09.88**

Die Akte enthält technische Angaben, die nach dem Eingang der Anmeldung eingereicht wurden und die nicht in dieser Patentschrift enthalten sind.

(51) Int. Cl.⁵: **A61K 31/565**, //(A61K31/565, 31:135)

(54) **Antigestagen- und antiöstrogenwirksame Verbindungen zur Behandlung hormonabhängiger Tumoren.**

(30) Priorität: **01.10.87 DE 3733478**

(43) Veröffentlichungstag der Anmeldung:
**05.04.89 Patentblatt 89/14**

(45) Bekanntmachung des Hinweises auf die Patenterteilung:
**08.06.94 Patentblatt 94/23**

(84) Benannte Vertragsstaaten:
**AT BE CH DE ES FR GB GR IT LI LU NL SE**

(56) Entgegenhaltungen:
**EP-A- 0 129 499**
**EP-A- 0 138 504**
**DE-A- 3 322 285**

(73) Patentinhaber: **SCHERING AKTIENGESELL-SCHAFT**

**D-13342 Berlin(DE)**

(72) Erfinder: **Schneider, Martin, Dr.**
**Fasanenstrasse 62**
**D-1000 Berlin 15(DE)**
Erfinder: **Elger, Walter, Dr.**
**Schorlemerallee 12B**
**D-1000 Berlin 33(DE)**
Erfinder: **Beier, Sybille, Dr.**
**Uhlandstrasse 121**
**D-1000 Berlin 31(DE)**
Erfinder: **Fähnrich, Marianne**
**Letteallee 48**
**D-1000 Berlin 51(DE)**
Erfinder: **Kosub, Beate**
**Forchbacher Strasse 51**
**D-1000 Berlin 37(DE)**
Erfinder: **Chwalisz, Krzysztof, Dr.**
**Luzerner Strasse 1d**
**D-1000 Berlin 45(DE)**
Erfinder: **Hasan, Syed Hamiduddin, Dr.**
**Angerburger Allee 37**
**D-1000 Berlin 19(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung mindestens einer Verbindung mit progesteron-antagonistischer (AG) und mindestens einer Verbindung mit antiöstrogener (AÖ) Wirkung in einem Gewichtsverhältnis von 1:50 bis 50:1 zur Herstellung eines Arzneimittels mit synergistischer Wirkung zur Behandlung hormonabhängiger Tumoren.

Antiöstrogenwirksame Verbindungen sind zur Behandlung von Krankheiten geeignet, die durch Östrogene bedingt oder von Östrogenen abhängig sind, beispielsweise zur Behandlung von östrogenabhängigen Tumoren, wie Mammakarzinom, Prostatahyperplasie oder Meningeom.

So wird zum Beispiel das Antiöstrogen Tamoxifen zur palliativen Behandlung des nichtoperablen Mammakarzinoms sowie zur adjuvanten Therapie nach Primärbehandlung des Mammakarzinoms angewandt. Mit Tamoxifen wird die Krankheit jedoch nicht geheilt. Für die Sekundärtherapie werden Gestagene oder Aromatasehemmer verwendet. In der Praemenopause führen Ovariektomie, Tamoxifen oder LHRH-Analoga (LHRH = Luteinizing hormon releasing hormons) zu vergleichbaren Ansprechraten (Lit. H.T. Mouridsen and R. Paridaens, Eur. J. Cancer Clin. Oncol., 24, S. 99 - 105, 1988).

In neuerer Zeit wird auch die Verwendung von Antigestagenen im Bereich der Tumortherapie, insbesondere für die Indikation Mammakarzinom diskutiert. Eine erste Phase-II-Studie mit $17\beta$-Hydroxy-$11\beta$-(4-dimethylaminophenyl)-$17\alpha$(prop-1-inyl)-estra-4,9-dien-3-on an postmenopausalen bzw. ovariektomierten Endokrintherapieresistenten Patientinnen mit metastasierendem Mammakarzinom wird von Maudelonde et al. in Hormonal Manipulation of Cancer, Eds. J.G.M. Klijn, R. Paridaens und J.A. Folkens in Raven Press, S. 55 (1987) berichtet.

Der Erfindung liegt die Aufgabe zugrunde. Arzneimittel für die Behandlung hormonabhängiger Tumoren bereitzustellen, die eine hohe, möglichst höhere Wirksamkeit im Vergleich zu den bekannten Mitteln haben.

Diese Aufgabe wird durch die Erfindung gelöst.

Es wurde gefunden, daß in der Kombination von AG und AÖ die Wirksamkeit der Einzelkomponenten beträchtlich verstärkt wird. Die erfindungsgemäße Kombination beruht auf der Erkenntnis, daß das Wachstum hormonabhängiger Tumoren gleichzeitig von Östrogenen und Gestagenen abhängig ist. So konnten in einem Großteil der Mammakarzinome sowohl Östrogen- als auch Progesteronrezeptoren nachgewiesen werden. Durch die Kombination von AG und AÖ auf Rezeptorebene im Tumor wird nicht nur eine Ovarblockade, sondern auch eine Blockade der aus anderen Geweben entstehenden betreffenden Hormone bewirkt. Eine Kombination von AG und AÖ eignet sich daher zur Therapie sowohl des prä- wie des postmenopausalen Mammakarzinoms.

Das Gewichtsverhältnis beider Komponenten kann dabei in weiten Grenzen variiert werden. So können sowohl gleiche Mengen AG und AÖ als auch ein Überschuß eines der beiden Komponente eingesetzt werden. AG und AÖ werden gemeinsam, getrennt, gleichzeitig und/oder zeitlich abgestuft (sequential), in einem Gewichtsverhältnis von im wesentlichen 1:50 bis 50:1, vorzugsweise 1:30 bis 30:1, und insbesondere 1:15 bis 15:1 verwendet.

Vorzugsweise können AG und AÖ kombiniert in einer Dosiseinheit appliziert werden.

Als Antigestagene kommen alle Verbindungen infrage, die eine starke Affinität zum Gestagenrezeptor (Progesteronrezeptor) besitzen und dabei keine eigene gestagene Aktivität zeigen. Als kompetitive Progesteronantagonisten kommen beispielsweise folgende Steroide infrage:

$11\beta$-[(4-N,N-Dimethylamino)-phenyl]-$17\beta$-hydroxy-$17\alpha$-propinyl-4,9(10)-estradien-3-on (RU-38486),

$11\beta$-[(4-N,N-Dimethylamino)-phenyl]-$17\beta$-hydroxy-18-methyl-$17\alpha$-propinyl-4,9(10)-estradien-3-on und

$11\beta$-[(4-N,N-Dimethylamino)-phenyl]-$17a\beta$-hydroxy-$17a\alpha$-propinyl-D-homo-4,9(10),16-estratrien-3-on (alle EP-A 0 057 115);

ferner

$11\beta$-p-Methoxyphenyl-$17\beta$-hydroxy-$17\alpha$-ethinyl-4,9(10)-estradien-3-on (Steroids 37 (1981) 361-382) und

$11\beta$-(4-Dimethylaminophenyl)-$17\alpha$-hydroxy-$17\beta$-(3-hydroxypropyl)-$13\alpha$-methyl-4,9-gonadien-3-on (EP-A 0129 499)

Die Antigestagene werden gemäß vorliegender Erfindung in Mengen von 10 mg bis 200 mg eingesetzt; im allgemeinen wird man mit 50 bis 100 mg $11\beta$-[(4-N,N-Dimethylamino)-phenyl]-$17\alpha$-hydroxy-$17\beta$-(3-hydroxypropyl)-$13\alpha$-methyl-4,9(10)-gonadien-3-on pro Tag oder einer biologisch äquivalenten Menge eines anderen Antigestagens auskommen.

Als antiöstrogen wirkende Verbindungen kommen Antiöstrogene und Aromatasehemmer infrage. Antiöstrogene und Aromatasehemmer gemäß vorliegender Erfindung können sowohl von Steroiden abgeleitet oder nicht-steroidale Verbindungen sein. Unter antiöstrogen wirkenden Verbindungen gemäß vorliegender Erfindung sollen aber nur solche Verbindungen verstanden werden, die möglichst selektiv wirken, d.h. die im wesentlichen nur die Wirkung von Östrogen hemmen und/oder deren Konzentration senken. Die Antiöstrogene wirken als kompetitive Östrogenantagonisten, indem sie Östrogen vom Rezeptor verdrängen, während Aromatasehemmer die Biosynthese des Östrogens unterdrücken. Verbindungen vom Typ des Aminoglutethimids, 3-alkylierte 3-(4-Aminophenyl)-piperidin-2,6-dione und andere, die außer dem Östrogenspiegel auch auf andere Sexualhormonserumkonzentrationen erniedrigend wirken, sind gemäß vorliegender Erfindung als antiöstrogen wirksame Verbindungen nicht geeignet.

Als Antiöstrogene kommen alle gebräuchlichen Antiöstrogene in Betracht, die vorstehend genannte Bedingung erfüllen. Sie können etwa in gleichen Mengen eingesetzt werden wie die bereits im Handel befindlichen Antiöstrogene, das heißt die tägliche Dosis beträgt etwa 5 - 100 mg für Tamoxifen oder biologisch äquivalente Mengen eines anderen Antiöstrogens. Als nicht-steroidale Antiöstrogene seien beispielsweise genannt:

Tamoxifen = (Z)-2-[p-(1,2-Diphenyl-1-butenyl)-phenoxy]-N,N-dimethyläthylamin,

Nafoxidin = 1-2-[4-(6-Methoxy-2-phenyl-3,4-dihydro-1-naphthyl)-phenoxy]-äthyl -pyrrolidin, hydrochlorid,

Mer 25 = 1-[p(2-Diäthylaminoäthoxy)-phenyl]-2-(p-methoxyphenyl)-1-phenyläthanol und

Verbindungen vom 1,1,2-Triphenylbut-1-en-Typ, insbesondere das 1,1-Bis(3'-acetoxyphenyl)-2-phenyl-but-1-en (J. Cancer Res. Clin. Oncol., (1986), 112, S. 119 - 124).

Ferner kommen als steroidale Antiöstrogene infrage:

$11\alpha$-Methoxy-$17\alpha$-äthinyl-1,3,5(10)-östratrien-3,$17\beta$-diol,

$16\beta$-Äthylestradiol und

11-(3,$17\beta$-Dihydroxy-1,3,5(10)-estratrien-$7\alpha$-yl)-undecansäure-(N-butyl-N-methyl) -amid (EP-A 0138 504).

Als Aromatasehemmer sind alle Verbindungen geeignet, die die Bildung von Östrogenen aus ihren Vorstufen hemmen, wie beispielsweise das in der deutschen Offenlegungsschrift 33 22 265 beschriebene 1-Methyl-androsta-1,4-dien-3,17-dion,

das in Journal of Clinical Endocrinology and Metabolism 49, 672 (1979) beschriebene

Testolacton (17a-Oxa-D-homoandrost-1,4-dien-3,17-dion),

die in "Endocrinology" 1973, Vol. 92, No. 3, Seite 874 beschriebenen Verbindungen

Androsta-4,6-dien-3,17-dion,

Androsta-4,6-dien-$17\beta$-ol-3-on-acetat.

Androsta-1,4,6-trien-3,17-dion,

4-Androsten-19-chlor-3,17-dion,

4-Androsten-3,6,17-trion,

die in der deutschen Offenlegungsschrift 31 24 780 beschriebenen

19-alkynylierten Steroide,

die in der deutschen Offenlegungsschrift 31 24 719 beschriebenen

10-(1,2-Propadienyl)-steroide.

die in der europäischen Patentanmeldung, Veröffentlichungsnummer 100 566 beschriebenen

19-Thio-androstanderivate,

das in "Endocrinology" 1977, Vol. 100, No. 6, Seite 1684 und der US-Patentschrift 4,235,893 beschriebene

4-Androsten-4-ol-3,17-dion und dessen Ester,

die in der deutschen Offenlegungsschrift 35 39 244 beschriebenen 1-Methyl-$15\alpha$-alkyl-androsta-1,4-dien-3,17-dione,

die in der deutschen Offenlegungsschrift 36 44 358 beschriebenen $10\beta$-Alkinyl-4,9(11)-östradien-derivate und das in der europäischen Patentanmeldung 0250262 beschriebene 1,$2\beta$-Methylen-6-methylen-4-androsten-3,17-dion.

Als nicht-steroidaler Aromatasehemmer sei beispielsweise das [4-(5,6,7,8-Tetrahydroimidazo[1,$5\alpha$]-pyri-din-5-yl)benzonitril-monohydrochlorid] erwähnt (Cancer Res., 48, S. 834-838, 1988).

Die Dosierung liegt bei 1 - 1000 mg 1-Methyl-androsta-1,4-dien-3,17-dion pro Tag oder biologisch äquivalenten Dosen von anderen Aromatasehemmern.

Antigestagen- und antiöstrogenwirksame Verbindungen können zum Beispiel lokal, topisch, subcutan, enteral oder parenteral appliziert werden.

Für die enterale Applikation kommen insbesondere Tabletten, Dragees, Kapseln, Pillen, Suspensionen oder Lösungen infrage, die in üblicher Weise mit den in der Galenik üblichen Zusätzen und Trägersubstanzen hergestellt werden können. Für die lokale oder topische Anwendung kommen beispielsweise Vaginalzäpfchen oder transdermale Systeme wie Hautpflaster infrage.

Die bevorzugte subcutane Injektion wird mit einer öligen Lösung der betreffenden Komponente (n) vorgenommen.

Eine AG-Dosiseinheit enthält etwa 10 - 200 mg $11\beta$-[(4-N,N-Dimethylamino)-phenyl]-$17\alpha$-hydroxy-$17\beta$-(3-hydroxypropyl)-$13\alpha$-methyl-4,9(10)gonadien-3-on oder eine biologisch äquivalente Menge eines anderen Antigestagens.

Eine AÖ-Dosiseinheit enthält 1 - 100 mg Tamoxifen oder 10 - 200 mg 1-Methylandrosta-1,4-dien-3,17-dion oder eine biologisch äquivalente Menge einer anderen antiöstrogen wirksamen Verbindung.

**Beispiel 1**

10,0 mg 11$\beta$-[(4-N,N-Dimethylamino)-phenyl]-17$\alpha$-hydroxy-17$\beta$-(3-hydroxypropyl)-13$\alpha$-methyl-4,9-gonadien-3-on
140,5 mg Laktose
69,5 mg Maisstärke
2,5 mg Polyvinylpyrrolidon 25
2,0 mg Aerosil
0.5 mg Magnesiumstearat
225,0 mg Gesamtgewicht der Tablette

**Beispiel 2**

50,0 mg 1-Methyl-androsta-1,4-dien-3,17-dion
115,0 mg Laktose
50,0 mg Maisstärke
2,5 mg Poly-N-Vinylpyrrolidon 25
2,0 mg Aerosil
0.5 mg Magnesiumstearat
220,0 mg Gesamtgewicht der Tablette

**Beispiel 3**

25,0 mg 1-Methyl-androsta-1,4-dien-3,17-dion
25,0 mg 11$\beta$-[(4-N,N-Dimethylamino)-phenyl]-17$\alpha$-hydroxy-17$\beta$-(3-hydroxypropyl)-13$\alpha$-methyl-4,9-gonadien-3-on
115,0 mg Laktose
50,0 mg Maisstärke
2,5 mg Poly-N-Vinylpyrrolidon 25
2,0 mg Aerosil
0.5 mg Magnesiumstearat
220,0 mg Gesamtgewicht der Tablette, die in üblicher Weise auf einer Tablettenpresse hergestellt wird. Gegebenenfalls können auch die erfindungsgemäßen Wirkstoffe mit jeweils der Hälfte der oben angegebenen Zusätze getrennt zu einer Zweischichtentablette gepreßt werden.

**Beispiel 4**

10,0 mg Tamoxifen
10,0 mg 11$\beta$-[(4-N,N-Dimethylamino)-phenyl]-17$\alpha$-hydroxy-17$\beta$-(3-hydroxypropyl)-13$\alpha$-methyl-4,9-gonadien-3-on
135,0 mg Laktose
60,0 mg Maisstärke
2,5 mg Poly-N-Vinylpyrrolidon 25
2.0 mg Aerosil
0.5 mg Magnesiumstearat
220.0 mg Gesamtgewicht der Tablette, die in üblicher Weise auf einer Tablettenpresse hergestellt wird. Gegebenenfalls können auch die erfindungsgemäßen Wirkstoffe mit jeweils der Hälfte der oben angegebenen Zusätze getrennt zu einer Zweischichtentablette gepreßt werden.

Die folgenden Beispiele 5 bis 12 beziehen sich auf die Zusammensetzungen öliger Lösungen. Die hergestellten Lösungen werden in Ampullen abgefüllt.

**Beispiel 5**

100,0 mg Tamoxifen
343,4 mg Rizinusöl
608.6 mg Benzylbenzoat
1052.0 mg  = 1 ml

**Beispiel 6**

55,0 mg 1-Methyl-androsta-1,4-dien-3,17-dion
55.0 mg 11$\beta$-[(4-N,N-Dimethylamino)-phenyl]-17$\alpha$-hydroxy-17$\beta$-(3-hydroxypropyl)-13$\alpha$-methyl-4,9-gonadien-3-on
343,4 mg Rizinusöl
608.6 mg Benzylbenzoat
1062,0 mg  = 1 ml

Die erfindungsgemäßen Wirkstoffe können auch mit jeweils der Hälfte der oben angegebenen Zusätze getrennt in zwei Kammern abgefüllt werden.

**Beispiel 7**

10 mg 11-(3,17$\beta$-Dihydroxy-1,3,5(10)estratrien-7$\alpha$-yl)-undecansäure-(N-butyl-N-methyl)-amid
0.9 ml Rizinusöl
0.1 ml Benzylbenzoat
1,0 ml

**Beispiel 8**

10 mg 11$\beta$-[(4-N,N-Dimethylamino)-phenyl]-17$\alpha$-hydroxy-17$\beta$-(3-hydroxypro pyl)-13$\alpha$-methyl -4,9-gonadien-3-on
0,9 ml Rizinusöl
0.1 ml Benzylbenzoat
1,0 ml

**Beispiel 9**

10 mg 1,1-Bis(3'-acetoxyphenyl)-2-phenyl-but-1-en
0.9 ml Olivenöl
1,0 ml

**Beispiel 10**

10 mg 11$\beta$-[(4N,N-Dimethylamino)-phenyl]-17$\beta$-hydroxy-17$\alpha$-(3-hydroxyprop-1(Z)-enyl)-4,9-estradien-3-on
0.9 ml Olivenöl
1,0 ml

**Beispiel 11**

60 mg 11-(3,17$\beta$-Dihydroxy-1,3,5(10)estratien-7$\alpha$-yl)-undecansäure-(N-butyl-N-methyl)-amid
10 mg 11$\beta$-[(4-N,N-Dimethylamino)-phenyl)-17$\alpha$-hydroxy-17$\beta$-(3-hydroxypropyl)-13$\alpha$-methyl -4,9-gonadien-3-on
0,9 ml Rizinusöl
0.1 ml Benzylbenzoat
1,0 ml

**Beispiel 12**

60 mg 1,1-Bis(3'-acetoxyphenyl)-2-phenyl-but-1-en
10 mg 11$\beta$-[(4-N,N-Dimethylamino)-phenyl]-17$\beta$-hydroxy-17$\alpha$-(3-hydroxyprop-1(Z)-enyl)-4,9-estradien-3-on

5

1.0 ml Olivenöl
1,0 ml

Ergebnisse

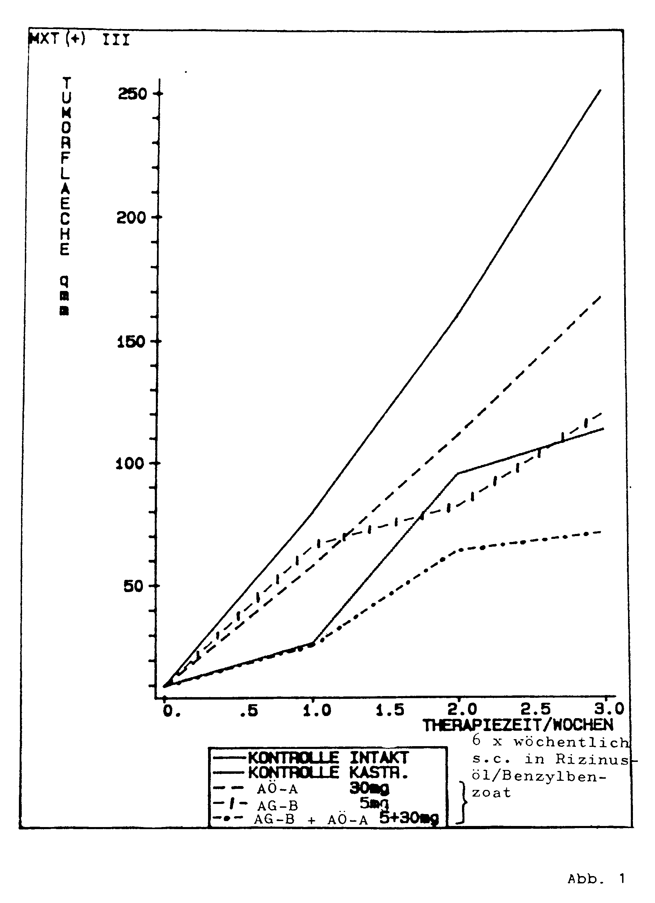

Die Ergebnisse der Untersuchungen am hormonabhängigen, östrogen- und progesteronrezeptor-positiven MXT (+)-Mammakarzinom der Maus (Watson C., Medina D., Clark J.H., Cancer Res. 1977 37, S. 3344-3348) sind den Tabellen 1 und 2 sowie der Abbildung 1 zu entnehmen.

Als MXT-Tumor wurde die XT-Linie M 3.2. verwendet, die freundlicherweise von Dr. A. E. Bogden, EG + G Bogden Laboratories, Worcester, MA, USA als gefrorene Probe zur Verfügung gestellt wurde. Nach Auftauen wurden Stücke mit einem Volumen von ungefähr 2 mm$^3$ subcutan in intakte, weibliche 8-10 Wochen alte BDF1-Mäuse (Charles River Wiga, BRD) implantiert.

Nachdem der Tumor einen Durchmesser von ungefähr 1 cm erreicht hatte, wurde er weiter auf BDF1-Mäuse übertragen, wie später beschrieben werden wird. Tumoren wurden von verschiedenen Generationen der Übertragungen genommen, eingefroren und in flüssigen Stickstoff aufbewahrt.

Für die Durchführung eines Versuches wurden Tumorstücke einer gefrorenen Probe in 3-5 Mäuse implantiert. Im nächsten Versuchsabschnitt wird die Hormonabhängigkeit der Tumoren durch Implantation in intakte und ovariektomierte Mäuse getestet (J. Med. Chem., 1985, 28, S. 1880-1885).

Wenn in den ovariektomierten Mäusen nach 6 Wochen eine Inhibierung des Tumorwachstums von mehr als 90 % im Vergleich zu den intakten Kontrolltieren auftritt, so können diese Tumoren für weitere Untersuchungen verwendet werden. Zwei bis drei Tumoren wurden von ein bis zwei als Spender dienenden Tieren entnommen und in MEM 199-Medium (MEM = Minimum Essential Medium) in Stücke von ungefähr 2 mm Durchmesser geschnitten. Diese Stücke werden - wie oben beschrieben - subcutan in BDF1-Mäuse (2 Tumoren / Maus) implantiert.

a) Therapie etablierter Tumoren

20 Tage nach Implantation der Tumoren werden die Mäuse nach Tumoren abgetastet. Nur Mäuse mit zwei ertastbaren Tumoren werden verwendet. Diese Tiere werden willkürlich in Gruppen von 9 bis 10 Tieren eingeteilt. Am nächsten Tag wird mit der 2 oder 3 Wochen dauernden Behandlung begonnen. Die Testsubstanzen werden 6 mal wöchentlich s.c. injiziert. Die Tumorflächen werden durch Messen mit einem Greifzirkel 1- oder 2mal wöchentlich gemessen. Als Tumorfläche gilt das Produkt aus dem längsten und dem dazu senkrechten Durchmesser. Am Ende der Behandlung werden die Tiere gewogen und getötet. Die Tumoren, Ovarien, Uteri und Vaginae wurden entfernt und deren Feuchtgewichte bestimmt (J. Med. Chem., loc. cit.).

b) Prophylaxe-Modell

Nach Implantation der Tumoren wurden die Tiere willkürlich in Gruppen von 9 - 10 Tieren eingeteilt. Am nächsten Tag wird mit der Behandlung begonnen. Die Testsubstanzen werden täglich subcutan als ölige Lösungen (10 %ige Benzylbenzoat-Lösung) injiziert, oder es wird eine Ovariektomie durchgeführt. Nach 6wöchiger Behandlung wird mit den Tieren wie oben weiterverfahren.

aa) Therapie etablierter Tumoren

Antiöstrogenwirksame Verbindungen

Eine 6 mal wöchentlich s.c. applizierte Dosis von 30 mg/kg Körpergewicht 11-(3,17$\beta$-Dihydroxy-1,3,5-(10)estratrien-7$\alpha$-yl)-undecansäure-(N-butyl-N-methyl)-amid (=AÖ-A) führte bei der Therapie etablierter Tumoren zu einer Wachstumshemmung von 33 % bezogen auf die Tumorfläche.

Antigestagene

Mit dem Antigestagen 11$\beta$-[(4-N,N-Dimethylamino)-phenyl]-17$\alpha$-hydroxy-17$\beta$-(3-hydroxypropyl)-13$\alpha$-methyl-4,9-gonadien-3-on (=AG-B) wurde bei s.c.-Applikation von 6 mal wöchentlich 5 mg/kg Körpergewicht eine Wachstumshemmung von 52 % bezogen auf die Tumorfläche beobachtet.

AG/AÖ-Kombination

Die Kombination der beiden Verbindungen AÖ-A und AG-B in den oben angegebenen Dosen verursacht eine Hemmung von 72 %, bezogen auf die Tumorfläche. Der Effekt der Kombination ist signifikant besser (p < 0,05) als die jeweiligen Monotherapien und ist sogar der Ovariektomie, wenn auch nicht signifikant, überlegen.

Werden zur Beurteilung der Wachstumshemmung der Tumoren nicht die Tumorflächen, sondern die Tumorgewichte herangezogen, gelangt man zu vergleichbaren Ergebnissen, wie sich aus Tabelle 1 ergibt.

bb) Prophylaktische Therapie von Tumoren (Tabelle 2)

Im Prophylaxe-Modell des MXT(+)-Tumors, bei dem die Therapie sofort nach der Implantation des Tumors begonnen und für 6 Wochen fortgesetzt wird, hat das Antiöstrogen 1,1-Bis-(3'-acetoxyphenyl)-2-phenyl-but-1-en (=AÖ-C) keinen signifikanten Antitumoreffekt (Dosis = 8 mg/kg)

Das Antigestagen $11\beta$-[(4-N,N-Dimethylamino)-phenyl]-$17\beta$-hydroxy-$17\alpha$-(3-hydroxyprop-1(Z)-enyl)-4,9-estradien-3-on (= AG-D) hemmt in diesem Modell das Tumorwachstum, und zwar um 68 %. Die Kombination der beiden vorstehend genannten Komponenten AÖ-C und AG-D führt ebenfalls zu einer deutlichen Verstärkung der Antitumorwirkung im Vergleich zu der antigestagen Komponente allein.

**T A B E L L E  1**    MXT(+)-MAMMAKARZINOM DER MAUS (THERAPIE ETABLIERTER TUMOREN)

| | Tumorfläche ($mm^2$) | % T/C | Tumorgewicht (mg) | % T/C |
|---|---|---|---|---|
| Kontrolle | 251 ± 134 | 100 | 2199 ± 1185 | 100 |
| Ovariektomie | 113 ± 61 | 45 | 941 ± 368* | 43 |
| AÖ-A, 30 mg/kg | 168 ± 41 | 67 | 1579 ± 389 | 72 |
| AG-B, 5 mg/kg | 120 ± 62 | 48 | 976 ± 513* | 44 |
| AÖ-A, 30 mg/kg + AG-B, 5 mg/kg | 71 ± 23 | 28 | 487 ± 153* | 22 |

*  $p < 0,05$ (U-Test) gegen Kontrolle

Dosierung: 6 x wöchentlich s.c. in Rizinusöl/Benzylbenzoat

EP 0 310 542 B1

TABELLE 2   EINFLUß VON AG-D ALLEIN UND IN KOMBINATION MIT AÖ-C MIT
MXT M 3.2 MAMMATUMOR-MODELL

| Substanz | Dosis (a) (mg/kg) | Tumorgewicht (b) (% T/C) |
|---|---|---|
| 1. AG-D | 1,0 | 32* |
| AG-D + AÖ-C | 1,0 + 8,0 | 8* |
| 2. AG-D | 1,0 | 47* |
| AG-D + AÖ-C | 1,0 + 16,0 | 21* |

(a)   Dosis: 3 x wöchentlich s.c. in Olivenöl
(b)   Werte nach 6 Wochen Therapie
% T/C   Therapiegruppe/Kontrolle x 100
*   $p < 0,01$ (U-Test nach Wilcoxon)

**Patentansprüche**

1. Verwendung mindestens einer Verbindung mit progesteron-antagonistischer (AG) und mindestens einer Verbindung mit antiöstrogener (AÖ) Wirkung in einem Gewichtsverhältnis von 1:50 bis 50:1 zur

9

Herstellung eines Arzneimittels mit synergistischer Wirkung zur Behandlung hormonabhängiger Tumoren.

2. Verwendung von AG und AÖ nach Anspruch 1 in einem Gewichtsverhältnis von 1:30 bis 30:1.

3. Verwendung von AG und AÖ nach Anspruch 1 in getrennten Dosiseinheiten.

4. Verwendung von AG und AÖ nach Anspruch 1 in einer gemeinsamen Dosiseinheit.

5. Verwendung von AG und AÖ nach Anspruch 1, dadurch gekennzeichnet, daß als AG 10 bis 200 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on oder eine biologisch äquivalente Menge einer anderen progesteron-antagonistisch wirksamen Verbindung in einer Dosiseinheit verwendet wird.

6. Verwendung von AG und AÖ nach Anspruch 1, dadurch gekennzeichnet, daß als AG 10 bis 200 mg 11$\beta$-[(4-N,N-Dimethylamino)-phenyl]-17$\beta$-hydroxy-17$\alpha$-(3-hydroxy-prop-1(Z)-enyl)-4,9-estradien-3-on in einer Dosiseinheit verwendet wird.

7. Verwendung von AG und AÖ nach Anspruch 1, dadurch gekennzeichnet, daß als AÖ 1 bis 100 mg Tamoxifen oder eine biologisch äquivalente Menge einer anderen antiöstrogen wirksamen Verbindung in einer Dosiseinheit verwendet wird.

8. Verwendung von AG und AÖ nach Anspruch 1, dadurch gekennzeichnet, daß als AÖ 10 bis 200 mg 1-Methyl-androsta-1,4-dien-3,17-dion oder eine biologisch äquivalente Menge einer anderen antiöstrogen wirksamen Verbindung in einer Dosiseinheit verwendet wird.

9. Verwendung von AG und AÖ nach Anspruch 1, dadurch gekennzeichnet, daß als AÖ 10 bis 200 mg 11-(3,17$\beta$-Dihydroxy-1,3,5(10)-estratrien-7$\alpha$-yl)-undecansäure-(N-butyl-N-methyl)-amid in einer Dosiseinheit verwendet wird.

10. Verwendung von AG und AÖ nach Anspruch 1, dadurch gekennzeichnet, daß als AÖ 10 bis 200 mg 1,1-Bis(3'-acetoxyphenyl)-2-phenyl-but-1-en in einer Dosiseinheit verwendet wird.

**Claims**

1. The use of at least one compound having progesterone-antagonistic (AG) activity and at least one compound having anti-oestrogenic (AO) activity in a weight ratio of from 1:50 to 50:1 for the manufacture of a medicament having a synergistic action for the treatment of hormone-dependent tumours.

2. The use of AG and AO according to claim 1 in a weight ratio of from 1:30 to 30:1.

3. The use of AG and AO according to claim 1 in separate dosage units.

4. The use of AG and AO according to claim 1 in a combined dosage unit.

5. The use of AG and AO according to claim 1, characterised in that from 10 to 200 mg of 11$\beta$-[(4-N,N-dimethylamino)-phenyl]-17$\alpha$-hydroxy-17$\beta$-(3-hydroxypropyl)-13$\alpha$-methyl-4,9(10)-gonadien-3-one or a biologically equivalent amount of another progesterone-antagonistic compound is used as AG in one dosage unit.

6. The use of AG and AO according to claim 1, characterised in that from 10 to 200 mg of 11$\beta$-[(4-N,N-dimethylamino)-phenyl]-17$\beta$-hydroxy-17$\alpha$-(3-hydroxy-prop-1(Z)-enyl)-4,9-oestradien-3-one is used as AG in one dosage unit.

7. The use of AG and AO according to claim 1, characterised in that from 1 to 100 mg of tamoxifen or a biologically equivalent amount of another anti-oestrogenically active compound is used as AO in one dosage unit.

**8.** The use of AG and AO according to claim 1, characterised in that from 10 to 200 mg of 1-methyl-androsta-1,4-diene-3,17-dione or a biologically equivalent amount of another anti-oestrogenically active compound is used as AO in one dosage unit.

**9.** The use of AG and AO according to claim 1, characterised in that from 10 to 200 mg of 11-(3,17$\beta$-dihydroxy-1,3,5(10)-oestratrien-7$\alpha$-yl)-undecanoic acid (N-butyl-N-methyl)-amide is used as AO in one dosage unit.

**10.** The use of AG and AO according to claim 1, characterised in that from 10 to 200 mg of 1,1-bis(3'-acetoxyphenyl)-2-phenyl-but-1-ene is used as AO in one dosage unit.

## Revendications

**1.** Emploi d'un ou plusieurs composés ayant une action antagoniste à l'égard de la progestérone (AG) avec un ou plusieurs composés ayant une action antioestrogène (AÖ), dans un rapport pondéral compris entre 1:50 et 50:1, pour préparer un médicament a effet de synergie en vue du traitement de tumeurs hormonodépendantes.

**2.** Emploi de AG et AÖ selon la revendication 1 dans un rapport pondéral compris entre 1:30 et 30:1.

**3.** Emploi de AG et AÖ selon la revendication 1 en doses unitaires séparées.

**4.** Emploi de AG et AÖ selon la revendication 1 en une dose unitaire commune.

**5.** Emploi de AG et AÖ selon la revendication 1, caractérisé en ce que l'on utilise comme AG de 10 à 200 mg de 11$\beta$[(4-N,N-diméthylamino)-phényl]-17$\alpha$-hydroxy-17$\beta$-(3-hydroxypropyl)-13$\alpha$-méthyl-4,9(10)-gonadién-3-one ou une quantité biologiquement équivalente d'un autre composé antagoniste de la progestérone en une dose unitaire.

**6.** Emploi de AG et AÖ selon la revendication 1, caractérisé en ce que l'on utilise comme AG de 10 à 200 mg de 11$\beta$-[(4-N,N-diméthylamino)-phényl]-17$\beta$-hydroxy-17$\alpha$-(3-hydroxy-prp-1(Z))-énye-4,9-estradién-3-one en une dose unitaire.

**7.** Emploi de AG et AÖ selon la revendication 1, caractérisé en ce que l'on utilise comme AÖ de 1 à 100 mg de tamoxifen ou une quantité biologiquement équivalente d'un autre composé antioestrogène, en une dose unitaire.

**8.** Emploi de AG et AÖ selon la revendication 1, caractérisé en ce que l'on utilise comme AÖ de 10 à 200 mg de 1-méthyl-androsta-1,4-diéne-3,17-dione ou une quantité biologiquement équivalente d'un autre composé antioestrogène, en une dose unitaire.

**9.** Emploi de AG et AÖ selon la revendication 1, caractérisé en ce que l'on utilise comme AÖ de 10 à 200 mg du (N-butyl-N-méthyl)-amide du 11-(3,17$\beta$-dihydroxy-1,3,5(10)-estratrién-7$\alpha$-yl)-undécanoïque en une dose unitaire.

**10.** Emploi de AG et AÖ selon la revendication 1, caractérisé en ce que l'on utilise comme AÖ de 10 à 200 mg de 1,1-bis(3'-acétoxyphényl)-2-phényl-but-1-ène en une dose unitaire.

Abb. 1